Europäisches Patentamt

**European Patent Office**

Office européen des brevets

(19)

(11) Numéro de publication : **0 046 130 B1**

(12) **FASCICULE DE BREVET EUROPÉEN**

(45) Date de publication du fascicule du brevet :
**21.11.85**

(51) Int. Cl.⁴ : **G 01 N   9/02, G 01 G  13/02**

(21) Numéro de dépôt : **81420122.4**

(22) Date de dépôt : **03.08.81**

(54) **Appareil permettant de déterminer automatiquement la masse par hectolitre de produits divers tels que les produits granuleux et les produits alimentaires; notamment les céréales et procédé de pesage de ces produits avec un tel appareil.**

(30) Priorité : **08.08.80 FR 8018081**
**08.05.81 FR 8109658**

(43) Date de publication de la demande :
**17.02.82 Bulletin 82/07**

(45) Mention de la délivrance du brevet :
**21.11.85 Bulletin 85/47**

(84) Etats contractants désignés :
**AT BE CH DE GB IT LI LU NL SE**

(56) Documents cités :
**DE-C-   728 668**
**FR-A-   796 336**
**FR-A- 1 019 068**
**FR-A- 1 288 120**
**FR-A- 2 389 130**
**FR-A- 2 436 983**

(73) Titulaire : **Magat, Georges**
**16 rue du Mai 8**
**F-42110 Feurs (FR)**

**Magat, Jean**
**8 rue du Mai 8**
**F-42110 Feurs (FR)**

(72) Inventeur : **Magat, Georges**
**16 rue du Mai 8**
**F-42110 Feurs (FR)**
Inventeur : **Magat, Jean**
**8 rue du Mai 8**
**F-42110 Feurs (FR)**

(74) Mandataire : **Dupuis, François**
**Cabinet Charras 3 Place de l'Hôtel-de-Ville**
**F-42000 St.Etienne (FR)**

Jouve, 18, rue St-Denis, 75001 Paris, France

## Description

La présente invention a pour objet un appareil tel que défini dans le préambule de la revendication 1. Elle a également pour objet un procédé tel que défini dans le préambule de la revendication 19.

Selon la technique actuelle, la mesure de la masse à l'hectolitre des céréales et autres produits granuleux, s'effectue de la façon suivante :

On remplit de produits une trémie conique située au-dessus d'une mesure établie et réglementée dans ses caractéristiques selon des normes. Après remplissage de la mesure et arasement de celle-ci, un opérateur la prend et la dispose manuellement sur une bascule, en vue d'établir le poids du contenu. Ce dernier est ensuite, par une nouvelle manutention, évacué dans des sacs ou autres, en vue d'une autre opération, puis l'opérateur positionne à nouveau la trémie sur la mesure vide, pour une nouvelle opération.

On comprend bien que ce mode de pesage présente plusieurs inconvénients.

A titre d'information, pour certains produits tels que le blé et autres céréales, et selon les normes, la mesure pèse 10 kilogrammes à vide et 50 kilogrammes en charge. Il est évident que la charge lourde à transporter de nombreuses fois, entraîne une fatigue certaine pour l'opérateur. Cette méthode est peu pratique, longue et pénible. De ce fait, on a constaté que certains opérateurs avaient tendance à apprécier eux-mêmes d'une manière subjective, les poids des charges, après quelques mesures effectives, avec évidemment une possibilité d'erreur non négligeable par rapport au poids spécifique réel, cette erreur étant multipliée par le nombre d'opérations.

Le but recherché selon l'invention était de mettre au point un appareil permettant de déterminer automatiquement la masse par hectolitre des céréales et autres produits granuleux, en réduisant au minimum les charges de l'opérateur à une fonction de contrôle et de mise en position de certains moyens, pour permettre l'opération automatique de pesage.

Un autre but selon l'invention, était de mettre au point un nouveau procédé de pesage des céréales et autres produits granuleux, indiquant directement la masse par l'hectolitre.

On connaît le Brevet FR-A-796 336 qui décrit un appareil permettant de déterminer automatiquement la masse volumique de produits divers, notamment les céréales, produits granuleux. Cet appareil comprend positionnés à l'intérieur d'un châssis, une balance, une mesure, une trémie ; la mesure et la trémie étant indépendantes. Cet appareil comprend un système particulièrement complexe de poids, contrepoids, certains étant fixes, d'autres mobiles. L'articulation entre ces divers éléments est complexe, agencée avec des moyens d'obturation partielle ou totale de la trémie de chargement, de la trémie compensatrice et de la benne. Le fonctionnement de cet appareil consiste à charger la benne et la trémie compensatrice jusqu'à un certain niveau indiqué par le contrepoids, puis il y a descente simultanée de ladite benne et de la trémie compensatrice, et à la suite d'un système complexe de commande, séparation de la benne par rapport à ladite trémie. La benne étant indépendante, elle agit en appui sur un moyen approprié permettant la lecture du poids de la charge dont l'évacuation s'effectue par l'ouverture du couvercle d'obturation situé dans le fond de la benne.

Les dispositions sont absolument incompatibles avec le mode d'utilisation selon le but recherché de la présente Demande.

Selon la revendication 1, l'invention a pour objet un appareil permettant de déterminer automatiquement la masse par hectolitre de produits divers tels que les produits granuleux et les produits alimentaires, notamment les céréales, comprenant une balance solidaire d'un châssis ainsi qu'une trémie de réception desdits produits et une mesure de référence positionnées indépendamment dans ledit châssis caractérisé en ce que ladite balance est disposée dans la partie supérieure du châssis et reçoit en appui sur son plateau, un étrier soutenant à sa partie inférieure ladite mesure de référence ; ladite trémie étant disposée entre ladite balance et ladite mesure de référence ; ledit appareil comprenant, en outre, des moyens de blocage de l'étrier ainsi que des moyens 12 autorisant le basculement de ladite mesure de référence.

Selon encore un autre but de l'invention, on a voulu étendre l'utilisation de l'appareil, par le prélèvement des produits granuleux, céréales et autres, directement lors de leur déversement de la trappe d'un véhicule dans la fosse de réception des produits.

Diverses variantes de l'appareil selon l'invention sont présentées dans les revendications dépendantes 2-18.

Selon la revendication 19, l'invention a également pour objet un procédé de pesage de produits granuleux par l'utilisation de l'appareil suivant les revendications 1 à 13. Ce procédé est caractérisé par un cycle opératoire comportant, d'une part, une première phase comprenant les étapes suivantes :

on bloque par verrouillage l'étrier supportant la mesure de référence, puis on procède au remplissage de la trémie de réception ;

on ouvre le volet d'obturation de ladite trémie pour permettre auxdits produits granuleux de tomber dans la mesure de référence et afin de remplir cette dernière ;

on procède à l'arasement de ladite mesure de référence au moyen dudit rouleau d'arasement lorsque cette mesure est pleine ; et, d'autre part, une deuxième phase comprenant les étapes suivantes :

on déverrouille l'étrier, on effectue ensuite la pesée du volume de produits granuleux contenu dans la mesure de référence, puis on procède à une nouvelle opération de verrouillage de l'étrier ;

on appuie sur ladite pédale de façon que le basculement communiqué vers l'avant à ladite mesure de référence entraîne l'escamotage dudit arceau de maintien en position de la mesure de référence ;

on relâche ladite pédale et on laisse la mesure de référence basculer vers l'arrière, autour dudit axe de rotation et sous l'effet de son poids, ce qui entraîne l'évacuation des produits granuleux pesés ;

on relève ladite mesure de référence lorsqu'elle est vide, puis on relève ledit arceau par action sur ladite chaînette afin que l'arceau assure à nouveau le maintien en position de ladite mesure de référence, en vue d'un nouveau cycle opératoire de pesée.

On souligne les avantages offerts par l'appareil et le procédé selon l'invention :

le nouveau procédé de pesage est simple, pratique et d'une grande efficacité ;

la trémie est indépendante par rapport au dispositif de pesage ;

on supprime totalement la manutention de force ;

la simplicité de l'appareil et son faible coût de revient ;

le traitement automatique et rapide, effectué directement lors du déversement de la trappe d'un véhicule.

Ces avantages et d'autres encore ressortiront bien de la suite de la description.

La figure 1 est une vue en perspective de l'appareil selon l'invention, avant remplissage de la mesure de référence, ci-après encore appelée mesure de référence.

La figure 2 est une vue de profil selon la figure 1.

La figure 3 est une vue de dessus en coupe verticale selon la ligne III-III de la figure 2, au-dessous de la trémie.

La figure 4 est une vue de dessus en coupe, selon la ligne IV-IV de la figure 2, au niveau de l'articulation de la mesure ;

La figure 5 est une vue de dessus en coupe, selon la ligne V-V de la figure 2, entre la trémie et la mesure ;

La figure 6 est une vue en perspective à plus grande échelle illustrant le verrouillage et le déverrouillage de l'étrier support de la mesure ;

La figure 7 est une vue illustrant une phase de fonctionnement de l'appareil avec le basculement vers l'avant de la mesure, pour la libérer de son élément de maintien, selon la commande de l'opérateur ;

La figure 8 est une vue illustrant la phase suivante de fonctionnement de l'appareil avec le basculement vers l'arrière de la mesure, pour l'évacuation des céréales pesées ;

La figure 9 est une variante de réalisation à grande échelle, du moyen de verrouillage ou de déverrouillage de l'étrier support de la mesure, ledit moyen étant en position de déverrouillage ;

La figure 10 est une vue selon la figure 9, le moyen étant en position de verrouillage ;

La figure 11 est à petite échelle, une vue en perspective de l'appareil équipé de l'alimenteur selon l'invention ; l'alimenteur est orienté perpendiculairement en regard du déversement des produits d'un véhicule, dans la fosse de réception, en vue d'obtenir un prélèvement. Le tracé en traits interrompus illustre l'alimenteur en position d'effacement ;

La figure 12 est une vue de profil en coupe transversale correspondant à la figure 11.

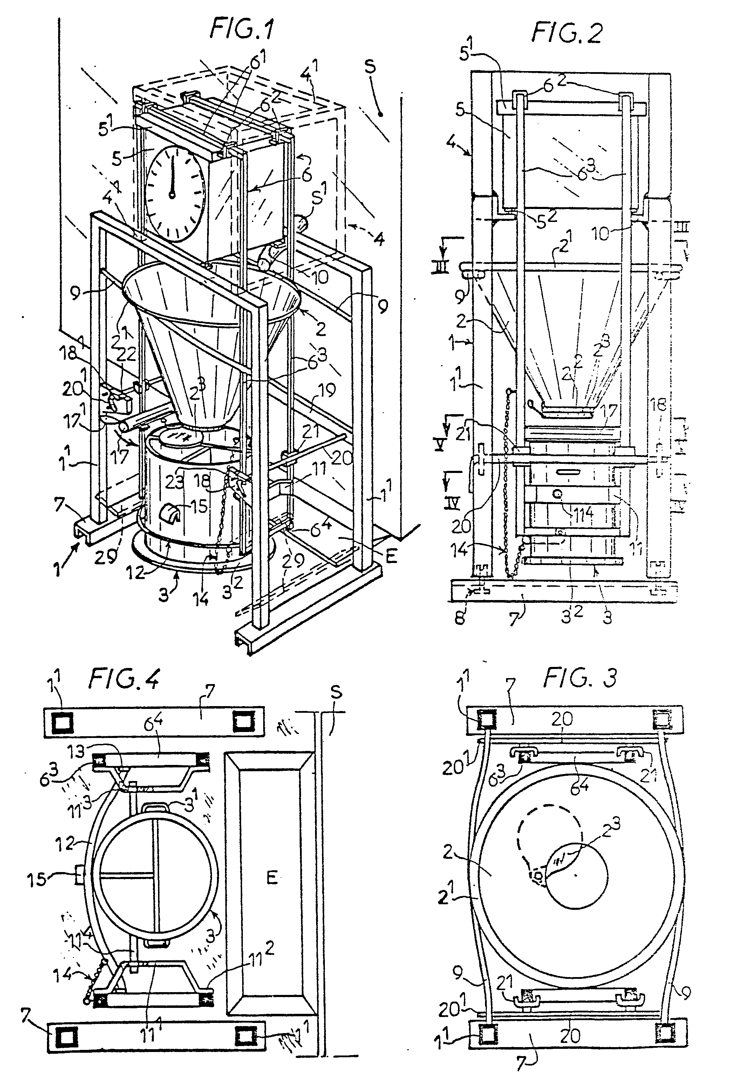

On a illustré figure 1, l'appareil en vue perspective. Cet appareil comprend cinq éléments principaux, à savoir : un châssis 1 à l'intérieur duquel sont positionnées indépendamment une trémie conique 2 et une mesure 3, un carter 4 de dimensions plus réduites, en appui superposé sur le châssis, une balance 5 fixée en appui sur la partie supérieure du châssis et protégée par le carter, un étrier 6 en appui sur le plateau $5^1$ de la balance 5 et soutenant à sa partie inférieure, la mesure 3 et la trémie conique 2 disposée de manière fixe sur le châssis 1. D'autres moyens importants apparaîtront dans la suite de la description.

Le châssis présente des moyens supportant la trémie conique et des moyens autorisant le blocage en position de l'étrier. Le châssis 1 est constitué de montants verticaux et horizontaux, de section carrée ou constitués de fers en U par exemple. Les extrémités inférieures des montants verticaux $1^1$ sont solidaires de traverses 7 profilées en U, au moyen d'éléments de fixation. Des vérins 8 assurent le réglage en hauteur du châssis. Ces montants verticaux $1^1$ sont convenablement entretoisés sur les côtés latéraux et le côté de fond dudit châssis 1. La trémie est supportée de manière définitive et indépendante de la mesure, par deux arceaux 9 ayant un profil courbé pour coopérer avec la bordure $2^1$ de la trémie 2. Ces arceaux 9 sont fixés à leurs extrémités sur les montants $1^1$ du châssis, par soudure ou autrement. La trémie présente dans son fond $2^2$, une ouverture avec un volet d'obturation $2^3$. A la partie supérieure du châssis 1, longitudinalement ou transversalement aux barres entretoises $1^2$, sont fixées des cornières en L 10, de dimensions sensiblement supérieures aux dimensions de la balance 5 dont le piètement $5^2$ vient en appui sur lesdites cornières. Un carter 4, de largeur plus réduite que le châssis 1, vient en appui sur ce dernier et a pour fonction de recouvrir et de protéger la balance 5. On dispose en appui sur le plateau $5^1$ de la balance, la partie supérieure de l'élément en forme d'étrier 6, ayant notamment pour fonction de porter la mesure 3. A cet effet, cet élément porteur est constitué de deux barres horizontales $6^1$ venant en appui et centrage, au moyen de plaquettes rapportées $6^2$, sur le plateau de la balance. Les plaquettes précitées $6^2$ sont fixées par soudure ou autrement sur les barres $6^1$. Ces barres horizontales $6^1$ se prolongent à leurs extrémités vers le bas, sous forme de barres verticales $6^3$, définissant de ce

fait un étrier. Une partie des barres verticales est sensiblement engagée à l'intérieur du profil des barres $4^1$ du carter 4, tandis que la partie inférieure se prolonge vers le bas en étant sensiblement éloignée de la bordure $2^1$ de la trémie conique, afin d'éviter tout contact avec elle. Des plaques profilées 11 sont rapportées et fixées à chacune de leurs extrémités, sur les barres $6^3$ de l'étrier 6. Chaque plaque présente une partie centrale déportée $11^1$, tandis que ses ailes $11^2$ présentent des ouvertures autorisant le passage de vis de fixation ou similaire, sur les barres précitées. En outre, dans la partie centrale $11^1$ est prévue une ouverture $11^3$ autorisant l'engagement et l'appui de deux axes $11^4$ solidaires de la mesure. Ces axes sont montés avec une liberté de rotation, dans les ouvertures précitées, permettant ainsi le basculement de la mesure ; un moyen empêche leur déplacement transversal. Ces axes $11^4$ sont rapportés et soudés contre les poignées de préhension $3^1$ de la mesure 3. D'une manière importante, comme il apparaît à la figure 4 des dessins, les deux axes sont déportés par rapport à l'axe médian transversal de ladite mesure. On comprend bien de ce fait, qu'il est nécessaire de prévoir un moyen de verrouillage en position verticale, de cette dernière ; ce moyen pouvant s'effacer pour permettre la vidange. La mesure présente de manière connue, une bordure périphérique $3^2$ définissant en quelque sorte, son piètement. Selon l'invention, on a prévu un autre arceau 12 profilé et escamotable sous l'action de l'opérateur. A cet effet, ledit arceau vient en appui par sa partie centrale, contre la mesure 3, tandis que les extrémités de l'arceau sont établies pour venir sensiblement le long des traverses inférieures $6^4$ de l'étrier. Sur chacune d'elles est prévu un doigt 13 sur lequel est engagée, à pivotement libre, l'extrémité $12^1$ de l'autre arceau 12. Un moyen assure le blocage en translation desdites extrémités. On a prévu en outre, une chaînette 14 dont les extrémités $14^1$ sont fixées sur ledit arceau lui-même et sur l'une des barres verticales de l'étrier, cette chaînette présentant une longueur suffisante permettant l'abaissement maximal de l'arceau par rapport à sa position initiale.

On a prévu sur le corps de la mesure, une poignée ou pédale 15 sur laquelle l'opérateur appuie dans une phase de fonctionnement bien précise, afin de dégager la mesure de l'arceau, assurant son blocage en position. Cette pédale 15 est située légèrement au-dessus par rapport au plan d'appui de l'arceau.

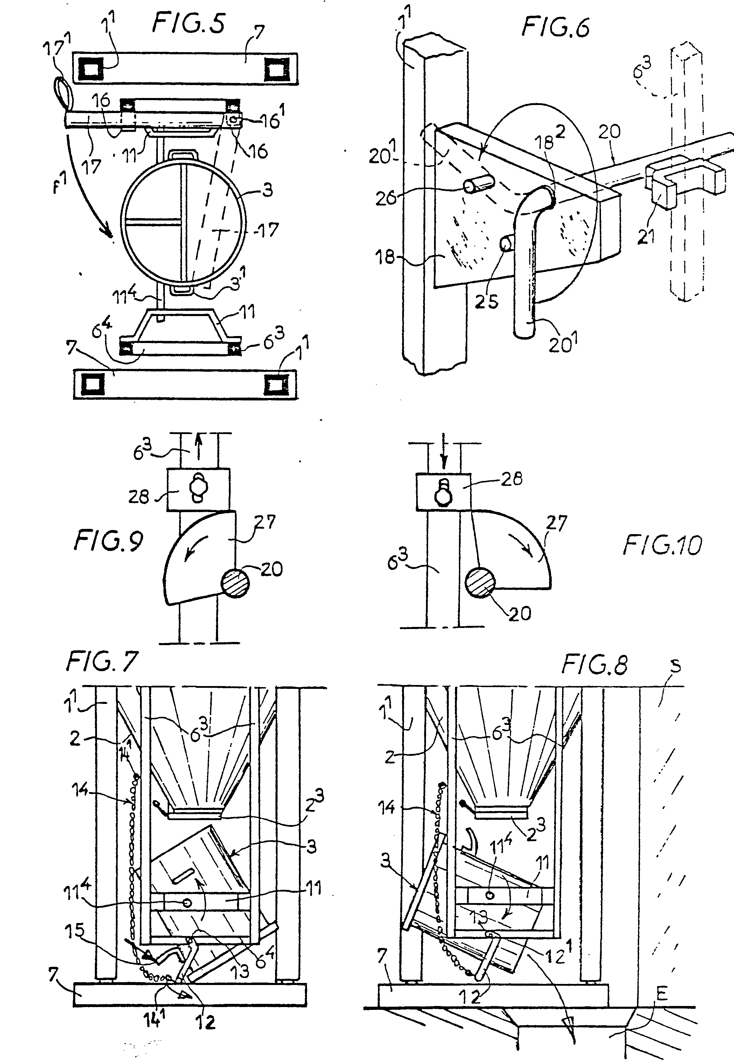

En outre, de la façon illustrée notamment aux figures 1 et 5, on prévoit sur les barres de l'étrier porte-mesure, au niveau du plan supérieur de celles-ci, deux pattes 16 profilées autorisant le positionnement d'un rouleau d'arasement 17. A cet effet, la patte 16 située sur la barre arrière de l'étrier, est munie d'un doigt $16^1$ dans lequel s'engage l'extrémité d'une patte prolongeant ledit rouleau, un moyen de blocage en translation étant prévu et rapporté sur le doigt, pour éviter l'enlèvement malencontreux du rouleau. En position de non-utilisation, ce dernier prend appui sur lesdites pattes, tandis qu'en cas d'utilisation avec préhension de sa poignée $17^1$, le rouleau pivote et se déplace transversalement autour de son axe de rotation, permettant ainsi l'arasement de la mesure.

Selon un autre aspect important de l'invention, on prévoit un dispositif de verrouillage ou de déverrouillage en position de l'étrier porte-mesure, afin de ne pas détériorer et fausser la bascule lors de la charge de la mesure et de sa vidange. Dans ce but, sur les montants verticaux $1^1$ du châssis, on dispose des consoles profilées 18 et entre les montants arrière, une traverse entretoise 19 située au même niveau que les consoles. Celles-ci présentent une échancrure $18^1$ dans laquelle est engagé en appui, un axe libre en rotation 20 support des éléments de verrouillage 21. Cet axe est engagé et fixé en libre rotation dans la traverse 19, un moyen empêchant le déplacement transversal dudit axe 20.

Selon une première réalisation, un cliquet 22 de verrouillage assure le positionnement stable dudit axe dans son logement. Cet axe 20 se prolonge à son extrémité, sous la forme d'une poignée de préhension $20^1$. En outre, il est prévu un doigt d'appui 23 sur chacune des consoles contre lequel, après pivotement, vient en butée la poignée de l'axe 20. Ledit axe présente en regard des barres de l'étrier lui faisant face, des profilés d'ancrage 21 de forme complémentaire à la section des barres 63, pour assurer leur maintien stable en position. Ces profilés d'ancrage peuvent se présenter sous la forme de pinces, de crochets ; ils peuvent se clipser en variante sur lesdites barres. Ils peuvent être en tout matériau plastique ou autre. En position de fermeture, la poignée $20^1$ de l'axe est orientée vers le bas, tandis que les profilés d'ancrage 21 sont ajustés étroitement sur les barres verticales de l'étrier, évitant ainsi toutes oscillations dans un plan horizontal, de ce dernier, par suite de la multiplication des points d'ancrage. En position ouverte, la poignée $20^1$ vient en appui sur le doigt 23, libérant ainsi lesdites barres des pinces d'ancrage ; on peut alors déterminer le poids en charge de la mesure. Il est bien évident que d'autres moyens de blocage peuvent être utilisés. Par exemple, comme illustré figure 6, les consoles 18 présentent des ouvertures $18^2$ autorisant l'engagement de l'axe 20 qui vient tantôt en butée contre un premier doigt 25, en position de verrouillage de l'étrier tantôt en butée contre un second doigt 26 en position de déverrouillage.

Comme illustré figures 9 et 10, on a prévu un moyen permettant le blocage en position de l'étrier 6, en évitant d'une part son oscillation sur le plan horizontal, et d'autre part, qu'il vienne en appui sur le plateau de la bascule pendant les phases de remplissage et de vidange. A cet effet, comme illustré, on prévoit sur l'axe 20 des cames 27 en regard de chaque barre verticale de l'étrier, lesdites cames étant fixées par tout moyen approprié tel que soudure.

En position illustrée figure 9, l'étrier est libre de telle façon que les cames 27 soient éloignées

desdites barres. Pour assurer le verrouillage des barres, on agit sur la poignée 20¹ en faisant pivoter l'axe. Les cames 27 pivotent également et viennent en appui progressif sur des butées 28 rapportées et fixées sur chaque barre précitée. Par leur action de pression sur la face inférieure des butées, les cames provoquent le soulèvement de l'étrier, supprimant ainsi tout poids sur la balance. On a prévu pour le réglage en position des butées sur les barres, des lumières dans lesquelles sont fixés les moyens de fixation. Par ce dispositif, on supprime donc à la fois l'oscillation de l'étrier dans le plan horizontal, et son appui sur le plateau de la balance, évitant ainsi de la fausser.

On a également prévu des déflecteurs 29 sur chacune des traverses horizontales de l'étrier et du châssis, afin de projeter les céréales au sol.

Il convient dès lors d'exposer le fonctionnement de l'appareil ;

Comme illustré figure 1, l'appareil est positionné près d'un silo à céréales, par exemple, S avec une bouche S1 autorisant l'arrivée des céréales dans la trémie conique. Une ouverture d'évacuation E est réalisée au sol, l'appareil venant s'engager autour d'elle.

La première phase est préalable au remplissage de la trémie ; on bloque en verrouillage l'étrier support de la mesure. Après cela, on remplit la trémie. Puis, par ouverture du volet d'obturation, les produits tombent dans la mesure. Lorsqu'elle est pleine, sans fermer le volet d'obturation, on arase la mesure au moyen du rouleau 17.

La phase suivante consiste à déverrouiller l'étrier 6 ; on effectue alors la lecture de la pesée en tenant compte ou non de la tare, selon le réglage préalable de la bascule. Une fois la lecture effectuée, on procède à une nouvelle opération de verrouillage de l'étrier. Puis l'opérateur appuie sur la pédale 15 en dégageant sa partie inférieure vers l'arrière, provoquant de ce fait un léger basculement vers l'avant de ladite mesure. L'arceau 12 de maintien en position de la mesure s'escamote. Par suite du départ de l'axe de rotation de la mesure et de par son poids, cette dernière, dans un mouvement inverse, bascule vers l'arrière, permettant ainsi l'évacuation des céréales pesées. Lorsque la mesure est vide, on la relève, puis il suffit de saisir la chaînette et de relever l'arceau 12 qui vient de nouveau en appui contre la mesure en la bloquant dans une position, en vue d'un nouveau cycle opératoire.

On expose maintenant un développement de l'invention, avec l'utilisation de l'appareil pour le prélèvement des produits granuleux, céréales ou autres, directement lors de leur déversement de la trappe d'un véhicule, dans la fosse de réception des produits.

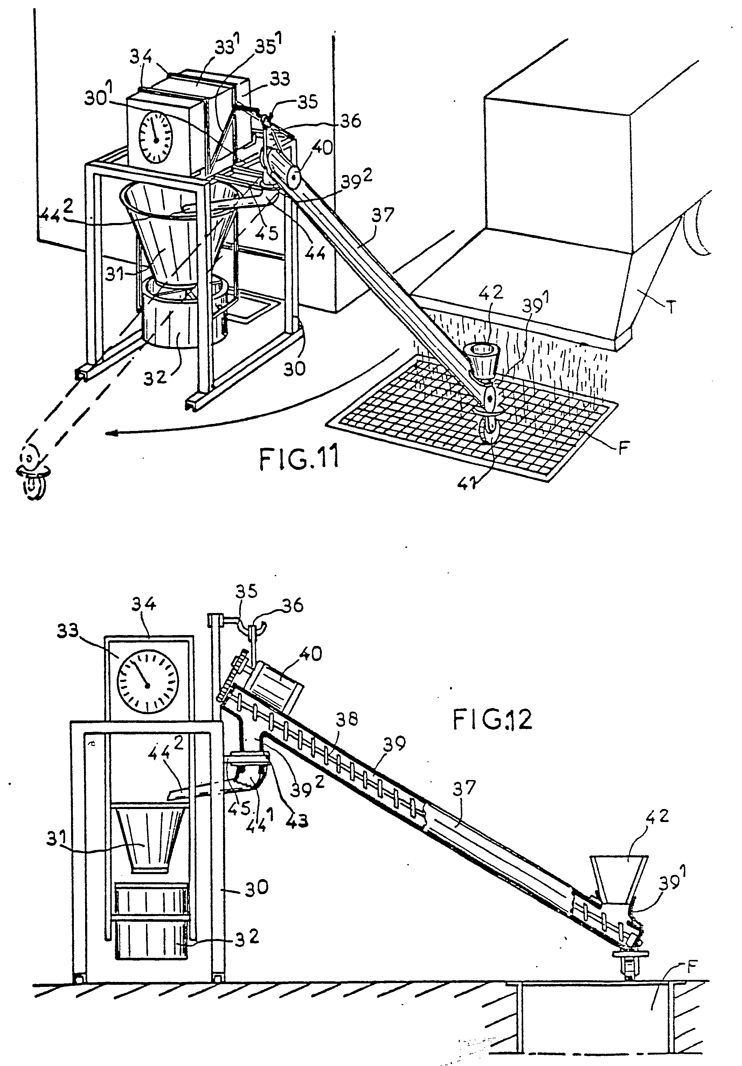

On voit à la figure 11, un appareil permettant de déterminer automatiquement la masse à l'hectolitre de produits divers, avec un châssis 30 à l'intérieur duquel sont positionnées indépendamment une trémie 31 de réception des produits et une mesure 32 de référence ; une balance 33 disposée dans la partie supérieure du châssis 30 reçoit sur son plateau 33¹ un étrier 34 supportant à sa partie inférieure, ladite mesure 32. Des moyens de blocage de l'étrier 34 sont également prévus, ainsi que des moyens autorisant le basculement de ladite mesure, après détermination de la masse desdits produits.

L'une des traverses supérieures latérales ou transversales 30¹ reliant les montants du châssis 30, permet en hauteur, la fixation d'une potence 35¹ munie à sa partie supérieure axiale, d'un doigt débordant formant crochet 35. Ce crochet coopère avec un anneau 36 de suspension solidaire de l'extrémité d'un alimenteur connu 37 à vis d'archimède 38 logé dans un corps tubulaire 39 et commandé en rotation par un moteur 40. Cet élévateur prenant à l'opposé appui au sol, par l'intermédiaire d'une ou de roulettes, ou galets 41, pivote ainsi, tant horizontalement qu'en hauteur, autour du crochet 35 de manière à se présenter soit dans une position transversale d'utilisation, comme illustré figure 11, soit dans une position latérale escamotée, tracée en traits interrompus.

La partie inférieure du corps 39 de l'alimenteur reçoit au droit de son ouverture supérieure d'arrivée 39¹ du produit, un réceptacle ouvert 42 en forme d'entonnoir, monté ou non à articulation pour se présenter horizontalement quel que soit le positionnement angulaire dudit alimenteur 37. Ce réceptacle 42 est destiné à se présenter dans la trajectoire de chute du produit, lors de son déversement de la trappe T d'un véhicule, dans la fosse de réception F.

La partie supérieure opposée du corps 39 reçoit au droit de son ouverture inférieure de sortie 39² du produit acheminé, un manchon rigide ou semi-rigide 43 fixé par emmanchement ou autrement, pour déborder vers le bas et se centrer librement dans l'ouverture supérieure correspondante 44¹ d'une trémie 44. Cette dernière est fixée transversalement sur la traverse 30¹ du châssis ou éventuellement sur ses montants, par l'intermédiaire d'un support profilé 45 formant collier, et constitue à sa base une goulotte coudée 44² de forme tronconique, présentant son orifice d'évacuation en regard de la partie axiale de l'ouverture supérieure de la trémie 31 inhérente à l'appareil.

Il est bien évident, selon ces dispositions, que l'alimenteur décrit peut être de tout autre type, notamment du type flexible, mais pouvant, dans tous le cas, fonctionner inversement pour évacuer, par l'orifice ouvert 39¹ de sa partie inférieure, dans la fosse de réception F, les produits contenus dans son corps 39, à seule fin d'éviter tout mélange en cas de prélèvements successifs de produits de natures différentes.

Lors du déversement des produits dans la fosse de réception F, par suite de l'ouverture de la trappe T du véhicule de transport, l'alimenteur 37 est déplacé manuellement de par sa roulette 41, pour pivoter autour du crochet 35, de manière à se présenter perpendiculairement et transversalement, comme illustré figure 11, compte tenu également de la dénivellation du sol et du positionnement en hauteur de l'appareil.

Le réceptacle ouvert 42 est placé horizontalement en regard de la chute des produits, pour assurer ainsi le prélèvement et l'acheminement par la mise en fonctionnement du moteur 40. Les produits se déversent ainsi de par le manchon 43, dans l'ouverture 44¹ et s'acheminent de par la goulotte 44², directement dans la trémie 31 de l'appareil, en vue de son remplissage après verrouillage de l'étrier-support de la mesure. Les phases d'utilisation de l'appareil restent ensuite conformes à celles décrites précédemment.

Après prélèvement et évacuation des produits pesés, directement ou indirectement dans la fosse de réception F, par pelletage ou autrement, le moteur 40 de l'alimenteur est mis en marche arrière pour permettre l'évacuation des produits non distribués, logés entre les spires de la vis d'entraînement 38 et en dehors de l'appareil.

Après utilisation, l'alimenteur 37 est pivoté pour s'escamoter latéralement par rapport à l'appareil, comme illustré par le tracé en traits interrompus de la figure 11.

L'appareil dans son ensemble peut être automatisé par toute commande appropriée, hydraulique, pneumatique ou électrique. Les automatismes peuvent être considérés au niveau de la relation de chacun des moyens dans leur relation fonctionnelle avec les autres moyens. On peut prévoir également un dispositif de lecture à distance de la masse à l'hectolitre, ainsi qu'une programmation électrique ou électronique, pour autoriser par exemple, le déplacement de l'alimenteur ; ce dernier peut également être commandé en translation par un moto-réducteur convenablement positionné.

## Revendications

1. Appareil permettant de déterminer automatiquement la masse par hectolitre de produits divers tels que les produits granuleux et les produits alimentaires, notamment les céréales, comprenant une balance (5, 33) solidaire d'un châssis (1) ainsi qu'une trémie (2, 31) de réception desdits produits et une mesure (3, 32) de référence positionnées indépendamment dans ledit châssis (1), caractérisé en ce que ladite balance (5, 33) est disposée dans la partie supérieure du châssis (1) et reçoit en appui sur son plateau (5¹, 33¹), un étrier (6, 34) soutenant à sa partie inférieure ladite mesure (3, 32) de référence ; ladite trémie (2, 31) étant disposée entre ladite balance (5, 33) et ladite mesure (3, 32) de référence ; ledit appareil comprenant, en outre, des moyens de blocage de l'étrier (6, 34) ainsi que des moyens (12) autorisant le basculement de ladite mesure (3, 32) de référence.

2. Appareil selon la revendication 1, caractérisé en ce que le châssis (1) comporte des moyens (20, 21, 27) autorisant le blocage en position de l'étrier (6, 34) ainsi que des moyens (9) supportant la trémie de réception (2, 31).

3. Appareil selon les revendications 1 et 2, caractérisé en ce que la trémie (2) présente une bordure (2¹) et est supportée par des arceaux (9) ayant un profil courbé pour coopérer avec la bordure (2¹) de ladite trémie, celle-ci présentant en outre, dans son fond, un volet d'obturation (2³).

4. Appareil selon la revendication 1, caractérisé en ce que l'on dispose en appui et centrage sur le plateau (5¹) de la balance (5), la partie supérieure (6¹) de l'étrier (6) qui se prolonge vers le bas sous forme de barres verticales (6³) sur lesquelles sont fixées à des niveaux différents, des moyens (11) supports de la mesure (3) de référence, des moyens (16) supports d'un rouleau d'arasement (17) et les moyens (12) autorisant le basculement de la mesure (3) de référence.

5. Appareil selon les revendications 1 et 4, caractérisé en ce que des plaques profilées (11) sont rapportées et fixées à chacune de leurs extrémités, sur les barres (6³) de l'étrier (6), chaque plaque présentant une partie centrale déportée (11¹) sur laquelle est prévue une ouverture (11³) autorisant l'engagement et l'appui de deux axes (11⁴) rapportés et fixés sur la mesure (3) de référence dont ils sont solidaires de poignées (3¹) de préhension ; lesdits axes étant montés avec une liberté de rotation.

6. Appareil selon les revendications 1, 4 et 5, caractérisé en ce que les axes (11⁴) sont déportés par rapport à l'axe médian transversal de la mesure (3) de référence.

7. Appareil selon les revendications 1, 4, 5 et 6, caractérisé en ce qu'un autre arceau (12) profilé et escamotable sous l'action d'un opérateur, vient en appui par sa partie centrale, contre la partie inférieure de la mesure (3) de référence, tandis que les extrémités (12¹) de l'arceau (12) sont établies pour venir sensiblement en appui le long de traverses inférieures (6⁴) de l'étrier (6) et s'engagent à pivotement libre sur un doigt (13) solidaire desdites traverses (6⁴).

8. Appareil selon la revendication 7, caractérisé en ce qu'une chaînette (14) relie par ses extrémités (14¹) l'autre arceau (12) et l'une des barres verticales (6³) de l'étrier, avec un certain degré de relâchement correspondant à l'abaissement maximal de l'arceau (12) par rapport à sa position initiale.

9. Appareil selon les revendications 1 et 7, caractérisé en ce qu'une pédale (15) est située légèrement au-dessus du plan d'appui de l'autre arceau (12) sur la mesure (3) de référence.

10. Appareil selon les revendications 1 et 4, caractérisé en ce qu'on prévoit sur les barres verticales (6³) de l'étrier (6), au niveau du plan d'ouverture de la mesure (3) de référence, deux pattes (16) profilées, autorisant le positionnement du rouleau d'arasement (17), l'une des pattes situées sur la barre arrière de l'étrier (6) étant munie d'un doigt (16¹) sur lequel s'engage l'extrémité d'une patte prolongeant ledit rouleau, en position de non-utilisation, ce dernier prenant appui sur les deux pattes (16).

11. Appareil selon les revendications 1 et 2, caractérisé en ce que les moyens de blocage de l'étrier (6) comprennent à partir des montants verticaux (1¹) du châssis, des consoles profilées (18) et entre les montants arrière, une traverse entretoise (19) située au même niveau que les consoles profilées (18), lesquelles sont aménagées pour recevoir à rotation libre, un axe (20) se prolongeant à son extrémité avant sous la forme d'une poignée de préhension (20¹) et étant muni en regard de chacune des barres verticales de l'étrier (6), de moyens de maintien de celles-ci ; un ou plusieurs doigts (23, 25, 26) étant disposés sur les consoles profilées (18) pour servir de butées à la poignée de l'axe (20), dans ses positions extrêmes.

12. Appareil selon les revendications 1, 2 et 11, caractérisé en ce que les moyens de maintien établis sur l'axe (20) sont des profils en U, en forme de pinces d'ancrage (21), enserrant la section des barres (6³) de l'étrier (6).

13. Appareil selon 1, 2 et 11, caractérisé en ce que les moyens de maintien établis sur l'axe (20) sont des cames (27) coopérant lors du pivotement de l'axe (20) avec des butées (28) établies en position réglable sur les barres de l'étrier (6), de manière à éviter toute oscillation dans un plan horizontal et en provoquant le soulèvement de l'étrier (6) par rapport au plateau de la balance.

14. Appareil selon la revendication 1, caractérisé en ce qu'il comprend un alimenteur mécanique entraîné par moteur (40) fixé en suspension articulée et déportée à la partie supérieure du châssis (30) et muni à sa base d'un moyen de roulement au sol (41), à seule fin d'autoriser son orientation par pivotement pour présenter perpendiculairement son orifice d'arrivée en regard de la chute des produits, lors de leur déversement du véhicule transporteur, dans la fosse (F) de réception ; l'orifice supérieur de sortie (39²) dudit alimenteur coopérant avec une trémie (44) en vue du déversement automatique desdits produits dans la trémie de réception (31) inhérente à l'appareil, pour obtenir ainsi un prélèvement de contrôle.

15. Appareil selon la revendication 14, caractérisé en ce que l'alimenteur à corps rigide ou flexible reçoit à sa base, au droit de son ouverture supérieure d'arrivée (39¹) des produits, un réceptacle ouvert (42) en forme d'entonnoir, fixé à demeure ou monté avec une possibilité de déplacement angulaire par rapport à l'axe longitudinal dudit alimenteur, de manière à présenter son axe dans la trajectoire de la chute des produits.

16. Appareil selon la revendication 14, caractérisé en ce que l'alimenteur reçoit à sa partie supérieure, un anneau de suspension et d'articulation (36) fixé sur son corps, pour s'engager sur un crochet (35) formé par un doigt débordant transversalement, supporté en surélévation par une potence (35¹) solidaire d'une des traverses supérieures latérales ou transversales (30¹) reliant les montants du châssis (30).

17. Appareil selon la revendication 14, caractérisé en ce que l'alimenteur reçoit à sa partie supérieure, au droit de son ouverture inférieure de sortie (39²) du produit, un manchon (43) qui s'engage librement dans l'ouverture supérieure correspondante d'une trémie déportée (44) fixée sur la traverse (30¹) du châssis par l'intermédiaire d'un support (45) et constituant à sa base une goulotte coudée et évasée (44²) dont l'orifice d'évacuation se présente en regard de la partie axiale de l'ouverture supérieure de la trémie de réception (31) inhérente à l'appareil.

18. Appareil selon les revendications 14, 15, 16 et 17, caractérisé en ce que l'alimenteur entraîné par moteur, agit en sens inverse, après l'opération de remplissage de la trémie de réception (31), pour autoriser l'évacuation extérieure du produit contenu dans son corps, en dehors de l'appareil.

19. Procédé de pesage de produits granuleux par l'utilisation de l'appareil selon les revendications 1 à 13, caractérisé par un cycle opératoire comportant, d'une part, une première phase comprenant les étapes suivantes :

on bloque par verrouillage l'étrier (6) supportant la mesure (3) de référence, puis on procède au remplissage de la trémie (2) de réception ;

on ouvre le volet d'obturation (2³) de ladite trémie (2) pour permettre auxdits produits granuleux de tomber dans la mesure (3) de référence et afin de remplir cette dernière (3) ;

on procède à l'arasement de ladite mesure (3) de référence au moyen dudit rouleau d'arasement (17) lorsque cette mesure est pleine ;

et, d'autre part, une deuxième phase comprenant les étapes suivantes :

on déverrouille l'étrier (6), on effectue ensuite la pesée du volume de produits granuleux contenu dans la mesure (3) de référence, puis on procède à une nouvelle opération de verrouillage de l'étrier (6) ;

on appuie sur ladite pédale (15) de façon que le basculement communiqué vers l'avant à ladite mesure (3) de référence entraîne l'escamotage dudit autre arceau (12) de maintien en position de la mesure (3) de référence ;

on relâche ladite pédale (15) et on laisse la mesure (3) de référence basculer vers l'arrière, autour dudit axe de rotation (11⁴) et sous l'effet de son poids, ce qui entraîne l'évacuation des produits granuleux pesés ;

on relève ladite mesure (3) de référence lorsqu'elle est vide, puis on relève ledit autre arceau (12) par action sur ladite chaînette (14) afin que l'arceau assure à nouveau le maintien en position de ladite mesure (3) de référence, en vue d'un nouveau cycle opératoire de pesée.

**Claims**

1. Apparatus for the automatic determination of the mass by the hectolitre of various products such as the granular products and the foodstuffs, more particularly the corn, including a balance (5, 33) integral with a frame (1) as well as a hopper (2, 31) for receiving said products and a reference measure (3, 32) positioned independently in said frame (1), characterized in that said balance (5, 33) is disposed within the upper part of the frame (1) and receives in abutment on the scale (5¹, 33¹) thereof a clevis (6, 34) supporting at the lower part thereof said reference measure (3, 32), said hopper (2, 31) being disposed between said balance (5, 33) and said reference measure (3, 32), said apparatus including moreover interlocking means for the clevis (6, 34) as well as the means (12) for tilting said reference measure (3, 32).

2. Apparatus as claimed in Claim 1, characterized in that the frame (1) is provided with means (20, 21, 27) for interlocking in position the clevis (6, 34) and with means (9) for supporting the receiving hopper (2, 31).

3. Apparatus as claimed in Claims 1 and 2, characterized in that the hopper (2) has a border (2$^1$) and is supported by small arches (9) having a curved contour for co-operating with the border (2$^1$) of said hopper, which is moreover provided at the bottom thereof with an obturator flap (2$^3$).

4. Apparatus as claimed in Claim 1, characterized in that there is disposed for abutment and centering on the scale (5$^1$) of the balance (5) the upper part (6$^1$) of the clevis (6) which is extended downwardly in the form of vertical bars (6$^3$) to which the following means are secured at various levels : the means (11) for supporting the reference measure (3) ; the means (16) for supporting a flush making roll (17), and the means (12) for tipping the reference measure (3).

5. Apparatus as claimed in Claims 1 and 4, characterized in that the contoured plates (11), at each one of the ends thereof, are inserted and secured onto the bars (6$^3$) of the clevis (6), each plate having an offset central portion (11$^1$) on which there is provided an opening (11$^3$) for the engagement and the abutment of two pins (11$^4$) inserted and secured onto the reference measure (3) and integral with gripping handles (3$^1$) thereof, said pins being mounted for free rotation.

6. Apparatus as claimed in Claims 1, 4 and 5, characterized in that the pins (11$^4$) are offset relative to the medial transverse axis of the reference measure (3).

7. Apparatus as claimed in Claims 1, 4, 5 and 6, characterized in that a further contoured small arch (12), collapsible under the action of an operator, come into abutment with the central portion thereof against the lower part of the reference measure (3), while the ends (12$^1$) of the small arch (12) are arranged for coming substantially into abutment along lower cross-bars (6$^4$) of the clevis (6) and engaged for free swinging on a finger (13) integral with said cross-bars (6$^4$).

8. Apparatus as claimed in Claim 7, characterized in that a small chain (14), by the ends (14$^1$) thereof, connects the other small arch (12) and one of the vertical bars (6$^3$) of the clevis, with some degree of relaxation corresponding to the maximal lowering of the small arch (12) relative to the initial position thereof.

9. Apparatus as claimed in Claims 1 and 7, characterized in that a pedal (15) is located slightly above the plane of abutment of the other small arch (12) on the reference measure (3).

10. Apparatus as claimed in Claims 1 and 4, characterized in that two contoured lugs (16) for positioning the flush making roll (17) are provided on the vertical bars (6$^3$) of the clevis (6), at the level of the opening plane of the reference measure (3), one of the lugs being located on the rear bar of the clevis (6) and provided with a finger (16$^1$) on which is engaged the end of a lug forming an extension of said roll in the inactive position thereof, said roll being abutted on the two lugs (16).

11. Apparatus as claimed in Claims 1 and 2, characterized in that the interlocking means of the clevis (6), from the vertical posts (1$^1$) of the frame, the contoured brackets (18), and between the rear posts, a bracing cross-bar (19) located at the same level as the contoured brackets (18), which are arranged for receiving for free rotation a pin (20) which is extended at the front end thereof in the form of a gripping handle (20$^1$) while being provided opposite each one of the vertical bars of the clevis (6) with holding means for these bars, one or more fingers (23, 25, 26) being disposed on the contoured brackets (18) to serve as stops for the handle of the pin (20) in the extreme positions thereof.

12. Apparatus as claimed in Claims 1, 2 and 11, characterized in that the holding means provided on the pin (20) are U-shaped contoured pieces in the form of anchoring clamps (21), encircling the section of the bars (6$^3$) of the clevis (6).

13. Apparatus as claimed in Claims 1, 2 and 11, characterized in that the holding means provided on the pin (20) are the cams (27), said cams, when the pin (20) is pivoted, co-operating with stops (28) disposed in an adjustable position on the bars of the clevis (6), in order to prevent any oscillation in an horizontal plane while causing the clevis (6) to be raised relative to the scale of the balance.

14. Apparatus as claimed in Claim 1, characterized in that said apparatus includes a mechanical feeder driven by a motor (40) secured by hinged and offset suspension to the upper part of the frame (30) and provided at its base with means (41) for rolling on the ground, for the sole purpose of the pivotal orientation thereof so that the arrival reception of this feeder will be presented normally to the chute of the products when the latter are delivered from the carriage vehicle into the reception pit (F), the upper outlet opening (39$^2$) of said feeder cooperating with a hopper (44) for the automatic pouring down of said products into the reception hopper (31) inherent to the apparatus, in order to obtain thereby that the products will be taken away by controlled amounts.

15. Apparatus as claimed in Claim 14, characterized in that the feeder with a rigid or flexible body is made to accommodate at the base thereof, at right angles to its upper arrival opening (39$^1$) of the products, an open funnel-shaped receptacle (42) which is mounted fixedly or with a possibility of angular shifting relative to the longitudinal axis of said feeder, so that the axis thereof will be presented within the plane of the chute of the products.

16. Apparatus as claimed in Claim 14, characterized in that the feeder is made to accommodate at the upper portion thereof a hanging and hinging ring (36) secured to the body of the feeder for engagement on a hook (35) which is formed by a transversely projecting finger, which is supported in a raised manner by a jib ($35^1$) integral with one of the upper lateral or transverse cross-pieces ($30^1$) connecting the posts of the frame (30).

17. Apparatus as claimed in Claim 14, characterized in that the feeder is made to accommodate at the upper portion thereof, at right angles to the lower outlet opening ($39^2$) of the product, a sleeve (43) which is engaged freely within the corresponding upper opening of an offset hopper (44) secured to the cross-piece ($30^1$) of the frame through the intermediary of a support (45) and constituting at the base thereof a bent and flared spout ($44^2$) having its discharge orifice opposite the axial portion of the upper opening of the reception hopper (31) inherent to the apparatus.

18. Apparatus as claimed in Claims 14, 15, 16 and 17, characterized in that the motor driven feeder is acting in the reverse direction, after the filling step of the reception hopper (31), in order to permit the external discharge of the product contained in the body of said feeder, outside the apparatus.

19. Method for weighing granulous products by using the apparatus claimed in Claims 1-13, characterized by an operational cycle including on the one hand a first phase comprising the following steps :

interlocking, by locking up the clevis (6) which supports the reference measure (3), then filling up of the reception hopper (2) ;

opening of the obturating flap ($2^3$) of said hopper (2) to permit said granular products to fall down into the reference measure (3), in order to fill up the latter ;

flush making of said reference measure (3) by means of said flush making roll (17) when this reference measure is filled up ;
and including on the other hand a second phase which comprises the following steps :

unlocking of the clevis (6), and then weighing of the volume of the granular products contained in the reference measure (3), and then a further interlocking step of the clevis (6) is carried out ;

depressing on said pedal (15) so that the tilting communicated forwardly to said reference measure (3) will caused said other small arch (12) for holding in position the reference measure (3) to be collapsed ;

release of said pedal (15) with the reference measure (3) being tipped rearwardly round said rotational axis ($11^4$) and under the effect of its own weight, the discharge of the weighed granular products being effected thereby ;

raising of said reference measure (3) when it is empty, then raising of said other small arch (12) by action on said small chain (14) so that this small arch will hold again in position said reference measure (3) in order to perform a further weighing operational cycle.

## Patentansprüche

1. Gerät zur automatischen Feststellung der hektolitermässige Menge von verschiedenen Produkten wie die Kornprodukte und die Nährmittelprodukte, insbesondere das Getreide, mit einer einem Rahmen (1) zugeordneten Waage (5, 33) sowie einem Trichter (2-31) für die Aufnahme der besagten Produkte und einem Bezugmass (3, 32), das unabhängig in dem genannten Rahmen (1) positioniert ist, dadurch gekennzeichnet, dass die genannte Waage (5, 33) in dem Oberteil des Rahmens (1) angeordnet ist und in Abstützung auf ihre Waageschale ($5^1$, $33^1$) einen Bügel (6, 34) aufnimmt, der an dessen Unterteil das genannte Bezugmass (3, 32) unterstützt ; dass der genannte Trichter (2, 31) zwischen der besagten Waage (5, 33) und dem genannten Bezugmass (3, 32) angeordnet ist ; und dass das besagte Gerät die Sperrmittel für den Bügel (6, 34) sowie die Mittel (12) darüber hinaus aufweist, die die Verschwenkung des genannten Bezugmasses (3, 32) gestatten.

2. Gerät nach Anspruch 1, dadurch gekennzeichnet, dass der Rahmen (1) die Mittel (20, 21, 27) für das Sperren des Bügels (6, 34) in seiner Stellung sowie die Mittel (9) für das Tragen des Aufnahmetrichters (2, 31) aufweist.

3. Gerät nach Anspruch 1 und 2, dadurch gekennzeichnet, dass der Trichter (2) eine Umrandung ($2^1$) aufweist und durch die Gewölbebögen (9) mit einer gekrümmten Formgebung zum Mitwirken mit der Umrandung ($2^1$) des besagten Trichters getragen ist, wobei dieser Trichter in dessen Boden eine Verschlussklappe ($2^3$) darüber hinaus aufweist.

4. Gerät nach Anspruch 1, dadurch gekennzeichnet, dass der Oberteil ($6^1$) des Bügels (6) abgestützt und zentriert auf der Waageschale ($5^1$) der Waage (5) angeordnet ist, der nach unten als Vertikalstangen ($6^3$) verlängert wird, auf welchen die Mittel (11) zum Tragen des Bezugmasses (3), die Mittel (16) zum Tragen einer Fluchtrolle (17) und die Mittel (12) für die Verschwenkung des Bezugmasses (3) auf verschiedene Höhen befestigt sind.

5. Gerät nach Anspruch 1 und 4, dadurch gekennzeichnet, dass profilierte Platten (11) an jeweiligem Ende derselben auf den Stangen ($6^3$) des Bügels (6) angebaut und befestigt sind, und jeweils einen versetzten, mittigen Teil ($11^1$) aufweisen, auf welchem eine Öffnung ($11^3$) für den Einsatz und die Abstützung zweier auf dem Bezugmass (3) angebauten und befestigten Achsen ($11^4$) vorgesehen ist, wobei die besagten Achsen den Handgriffen ($3^1$) zugeordnet und mit Drehbewegungsfreiheit montiert sind.

6. Gerät nach Anspruch 1, 4 und 5, dadurch gekennzeichnet, dass die Achsen ($11^4$) in bezug auf die mittige Querachse des Bezugmasses (3), versetzt sind.

7. Gerät nach Anspruch 1, 4, 5 und 6, dadurch gekennzeichnet, dass ein anderer Gewölbebogen (12), profiliert und zurückklappbar unter der Einwirkung eines Arbeiters, durch seinen mittigen Teil in Abstützung gegen den Unterteil des Bezugmasses (3) kommt, während die Enden (12¹) des Gewölbebogens (12) für fühlbare Abstützung den Niederquerstücken (6⁴) des Bügels (6) entlang vorgesehen sind und auf einen den genannten Querstücken (6⁴) zugeordneten Finger (13) mit freier Verschwenkbewegung eingreifen.

8. Gerät nach Anspruch 7, dadurch gekennzeichnet, dass der andere Gewölbebogen (12) durch dessen Enden (14¹) und die eine der Vertikalstangen (6³) des Bügels mittels eines Kettchens (14) mit einem gewissen Grad an Lockerung verbunden werden, wobei der Wert der Lockerung der höchsten Senkung des Gewölbebogens (12) in bezug auf die Anfangsstellung desselben entspricht.

9. Gerät nach Anspruch 1 und 7, dadurch gekennzeichnet, dass ein Fusshebel (15) etwa oberhalb der Abstützungsebene des anderen Gewölbebogens (12) auf dem Bezugsmass (3) vorgesehen ist.

10. Gerät nach Anspruch 1 und 4, dadurch gekennzeichnet, dass zwei profilierte Laschen (16) für die Positionierung der Fluchtrolle (17) auf den Vertikalstangen (6³) des Bügels (6) an der Höhe der Öffnungsebene des Bezugmasses (3) vorgesehen sind, wobei die eine der auf der Hinterstange des Bügels (6) befindlichen Laschen mit einem Finger (16¹) versehen ist, auf welchem das Ende einer, die besagte Rolle verlängernden Lasche, bei Abstützen dieser Rolle auf die zwei Laschen (16), in der Ruhestellung eingreift.

11. Gerät nach Anspruch 1 und 2, dadurch gekennzeichnet, dass die Sperrmittel für den Bügel (6) die folgenden Bauteile aus den Vertikalständern (1¹) des Rahmens aufweist : die profilierten Konsolen (18) ; ein zwischen den Hinterständern und an derselben Höhe wie die profilierten Konsolen (18) befindliches Querstück (19), wobei die besagten Konsolen zur Aufnahme mit freier Drehbewegung einer Achse (20) eingerichtet sind, die an derer Vorderende als Handgriff (20¹) verlängert wird und entgegengesetzt jeder Vertikalstange des Bügels (6) mit Haltemitteln für diese Stangen versehen ist ; einen oder mehrere auf den profilierten Konsolen (18) befindlichen Finger (23, 25, 26), die auf den profilierten Konsolen (18) vorgesehen sind, um als Anschläge für den Handgriff der Achse (20) in den äussersten Stellungen derselben zu dienen.

12. Gerät nach Anspruch 1, 2 und 11, dadurch gekennzeichnet, dass die auf der Achse (20) vorgesehenen Haltemittel aus U-förmigen, als Verankerungszangen (21) ausgestalteten Profilierstücken bestehen, die den Querschnitt der Stangen (6³) des Bügels (6) einschliessen.

13. Gerät nach Anspruch 1, 2 und 11, dadurch gekennzeichnet, dass die auf der Achse (20) vorgesehenen Haltemittel aus Kurven (27) bestehen, die bei der Verschwenkung der Achse (20) mit auf die Stangen des Bügels (6) verstellbar angeordneten Anschlägen (28) mitwirken, damit jede Schwingung in der waagrechten Ebene vermieden wird, wobei das Anheben des Bügels (6) in bezug auf die Waageschale der Waage entsteht.

14. Gerät nach Anspruch 1, dadurch gekennzeichnet, dass dieses Gerät eine Zufuhrvorrichtung aufweist, die durch einen Motor (40) angetrieben wird, der mit am Oberende des Rahmens gelenkiger und versetzter Aufhängung befestigt und am Fuss desselben mit einem Mittel zum Rollen auf den Boden (41) versehen ist, damit die Orientierung durch Verschwenkung der Zufuhrvorrichtung mit senkrechter Vorlage der Eintrittsöffnungen derselben entgegengesetzt dem Niederfallen der Produkte bei dem Ausschütten derselben aus dem Beförderungsfahrzeug in die Aufnahmegrube (F) ermöglicht wird, wobei die obere Austrittsöffnung (39²) der besagten Zufuhrvorrichtung mit einem Trichter (44) für das automatische Ausschütten der genannten Produkte in dem dem Gerät zugeordneten Aufnahmetrichter (31) zusammenwirkt, um somit eine Kontrollentnahme zu erreichen.

15. Gerät nach Anspruch 14, dadurch gekennzeichnet, dass die Zufuhrvorrichtung mit starrem oder biegsamen Gehäuse, am Fuss derselben und rechtwinklig zu ihrer oberen Öffnung (39¹) für das Eintreffen der Produkte, einen geöffneten, trichterförmigen Sammelbecken (42) aufnimmt, der ortsfest oder mit Möglichkeit einer winkligen Verschiebung in bezug auf die längliche Achse der besagten Zufuhrvorrichtung vorgesehen ist, damit ihre Achse im Fallweg der Produkte vorgelegt wird.

16. Gerät nach Anspruch 14, dadurch gekennzeichnet, dass die Zufuhrvorrichtung, am Oberteil derselben, einen auf ihrem Gehäuse befestigten Aufhängungs- und Gelenkungsring (36) zum Eingreifen auf einen Haken (35) aufnimmt, der auf einem querseitig herausragenden Finger gebildet ist, der hochliegend von einem mit dem einen der seitlichen oder querseitigen, die Ständer des Rahmens (30) verbindenden Oberquerstücke (30¹) festgemachten Ausleger (35¹) getragen wird.

17. Gerät nach Anspruch 14, dadurch gekennzeichnet, dass die Zufuhrvorrichtung, am Oberteil derselben, rechtwinkling zu ihrer unteren Öffnung (39²) für den Austritt des Produkts, eine Muffe (43) für freien Einsatz in die entsprechende Oberöffnung eines versetzten, auf dem Querstück (30¹) des Rahmens mittels eines Trägers (45) befestigten Trichters (44) aufnimmt, der am Fuss desselben eine gekrümmte und ausgeweitete Rinne (44²) bildet, derer Entleerungsöffnung entgegengesetzt dem mittigen Teil der Oberöffnung des Empfangstrichters (31) liegt, der dem Gerät anhaftet.

18. Gerät nach Anspruch 14, 15, 16 und 17, dadurch gekennzeichnet, dass die durch Motor angetriebene Zufuhrvorrichtung in umgekehrter Richtung nach dem Abfüllvorgang des Empfangstrichters (31) wirkt, um das aussenseitige Entlassen des in dem Gehäuse der Zufuhrvorrichtung enthaltenen Produkts aus dem Gerät zu gestatten.

19. Verfahren zum Abwiegen von Kornprodukten durch Verwendung des Geräts nach Anspruch 1 bis 13, dadurch gekennzeichnet, dass der Arbeitsablauf eine erste Durchführungsstufe mit den folgenden Vorgängen erfasst :

Absperren durch Verriegelung des das Bezugmass (3) tragenden Bügels (6), und dann Abfüllen des Empfangstrichters (2) ;

Öffnung der Schliessklappe (2³) des besagten Trichters (2), damit die körnigen Produkte in das Bezugmass (3) niederfallen können, und damit dieses Bezugmass (3) abgefüllt wird ;

Fluchtmachen, mittels der besagten Fluchtrolle (17), des genannten Bezugmasses (3), wenn letzteres voll abgefüllt ist ;

und dass der vorerwähnte Arbeitsablauf eine zweite Durchführungsstufe mit den folgenden Vorgängen erfasst :

Entriegelung des Bügels (6), Abwiegen der Menge der in dem Bezugmass (3) enthaltenen körnigen Produkte, und dann erneute Verriegelung des Bügels (6) ;

Andruck auf den besagten Fusshebel (15), damit die nach dem Vorderende des genannten Bezugmasses (3) gerichtete Verschwenkung das Zurückklappen des besagten Gewölbebogens (12) zum Aufhalten in Stellung des Bezugmasses (3) bewirkt ;

Loslassen des besagten Fusshebels (15) mit Rückschwenkung des Bezugmasses (3) um die besagte Drehachse (11⁴) herum unter der Einwirkung des Eigengewichts dieses Bezugmasses, wobei die abgewogenen körnigen Produkte entleert werden ;

Anheben des entleerten Bezugmasses (3) und dann des genannten Gewölbebogens (12) durch Einwirkung auf das genannte Kettchen (14), damit das Aufhalten in Stellung des genannten Bezugmasses (3) für einen weiteren Abwiegevorgang von dem Gewölbebogen erneut bewirkt wird.

FIG.1

FIG.2

FIG.4

FIG. 3

0 046 130

FIG.5

FIG.6

FIG.9

FIG.10

FIG.7

FIG.8

FIG.11

FIG.12